# EUROPÄISCHE PATENTSCHRIFT

(11) **EP 2 262 457 B1**
(45) Veröffentlichungstag und Bekanntmachung des Hinweises auf die Patenterteilung: **28.09.2011**
(21) Anmeldenummer: 09707107.0
(22) Anmeldetag: 11.03.2009
(51) Int. Cl.: A61F 9/00

(54) **OPHTHALMOCHIRURGISCHES SYSTEM**
OPTHALMOSURGICAL SYSTEM
SYSTÈME POUR CHIRURGIE OPHTALMOLOGIQUE

(30) Priorität: 12.03.2008 DE 102008013950
(43) Veröffentlichungstag der Anmeldung: 22.12.2010
(73) Patentinhaber: Carl Zeiss Surgical GmbH, 73447 Oberkochen (DE)
(72) Erfinder: EICHLER, Michael, Dr.-Ing., 73434 Aalen (DE)
(74) Vertreter: Gnatzig, Klaus
(86) Internationale Anmeldenummer: PCT/EP2009/001747
(87) Internationale Veröffentlichungsnummer: WO 2009/112251

(56) Entgegenhaltungen:
- EP-A- 0 717 970
- US-A- 4 369 785
- US-A- 5 342 293
- US-A- 5 649 905

## Beschreibung

Die Erfindung betrifft ein ophthalmochirurgisches System, vorzugsweise zur Durchführung einer Phakoemulsifikation.

Zur Behandlung einer Linsentrübung, welche als Grauer Star oder Katarakt bezeichnet wird, gibt es mehrere chirurgische Techniken. Die am weitesten verbreitete Technik ist die Phakoemulsifikation, bei der ein dünnes Rohr in Form einer Hohlnadel in die Augenlinse eingeführt und mit Ultraschall zu Schwingungen angeregt wird. Die vibrierende Nadel emulsifiziert in ihrer nächsten Umgebung die Linse derart, dass die entstehenden Linsenfragmente durch eine Leitung von einer Pumpe abgesaugt werden können. Ist die Linse vollständig emulsifiziert worden, kann in den leeren Kapselsack eine neue künstliche Linse eingesetzt werden, so dass ein derart behandelter Patient wieder ein gutes Sehrvermögen erreicht.

Bei der Phakoemulsifikation kommt eine Vorrichtung zum Einsatz, welche allgemein eine Quelle mit Irrigationsfluid, eine Irrigationsfluidleitung für die Zufuhr von Irrigationsfluid zu der zu behandelnden Augenlinse, eine schwingfähige Nadel in einem Handstück und eine Saugleitung (Aspirationsleitung) zum Abtransportieren emulsifizierter Linsenfragmente in einen Sammelbehälter aufweist.

Die Irrigationsfluidquelle ist höher als die zu behandelnde Augenlinse angeordnet, so dass das Irrigationsfluid oder Spülfluid mit einem hydrostatischen Druck zum Auge fließen kann. Die Irrigationsfluidquelle lässt sich üblicherweise je nach Anwendungsfall in unterschiedliche Höhen verlagern, so dass ein unterschiedlicher hydrostatischer Druck erreichbar ist. Dazu ist die Irrigationsfluidquelle an einer Stange aufgehängt, welche mit einem Motor angetrieben werden kann und somit unterschiedliche Höhen erreicht. Ein derartiges Verlagern der Irrigationsfluidquelle benötigt einige Sekunden, bis die gewünschte Höhe erreicht ist. Ferner muss abgewartet werden, bis Druckschwankungen, die durch die Verlagerung der Irrigationsfluidquelle induziert wurden, abgeklungen sind, bevor eine Operation fortgesetzt werden kann. Die Höhe der Verlagerung einer solchen Quelle ist aufgrund der Stange mechanisch begrenzt. Der maximale hydrostatische Druck des Irrigationsfluids ist somit durch die erreichbare Höhe der Irrigationsfluidquelle vorgegeben.

Aus der EP 0 717 970 A1 ist eine ophthalmologische Aspirations- und Irrigationseinrichtung bekannt, die bei der Durchführung mikrochirurgischer Eingriffe am Auge eines Lebewesens zum Einsatz kommt. Das System umfasst eine Infusionseinheit mit zwei miteinander verbundenen Behältern für eine Salzlösung. Die beiden Behälter sind dabei derart miteinander verschaltet, dass Salzlösung aus dem ersten Behälter in den zweiten Behälter tropft, wobei das Niveau der Salzlösung im Innenraum des zweiten Behälters durch die Zuführung der Salzlösung aus dem ersten Behälter konstant gehalten werden kann. Aus dem zweiten Behälter ist die Salzlösung über eine Irrigationseinheit und eine Druckeinheit dem Auge zuführbar. Das ophthalmochirurgische System umfasst ferner eine Aspirationseinheit mit einer Aspirationsfluidleitung und eine Pumpeneinheit, mit deren Hilfe Fluid von dem Auge abführbar ist.

In der Praxis hat sich gezeigt, dass unterschiedliche Operationen am Auge auch unterschiedliche Fluiddrücke erfordern. Eine Operation am Augenvorderabschnitt verlangt einen anderen Druck als eine Operation am Augenhinterabschnitt. Zudem sind bei unterschiedlichen Operationen auch jeweils unterschiedliche Bestecke wie z.B. Nadeln mit unterschiedlichen Durchmessern für ein Phakoemulsifikationshandstück erforderlich, wobei mit verschiedenen Drücken gearbeitet werden muss. Die bei derzeit verfügbaren Systemen für die Phakoemulsifikation erreichbaren Fluiddrücke reichen für derartige Anwendungen oft nicht aus.

Es besteht ferner ein Trend in der minimalinvasiven Chirurgie, dass eine Inzision mit immer kleineren Instrumenten bzw. Nadeln durchgeführt wird. Die Ursache für diesen Trend liegt hauptsächlich darin, dass eine postoperative Behandlung eines Patienten umso seltener durchgeführt werden muss, je kleiner der bei der Operation durchgeführte Schnitt ist. Bei einem sehr kleinen Schnitt muss keine Nachbehandlung durchgeführt werden, so dass keine weiteren Komplikationen mehr auftreten können. Der Einsatz von Hohlnadeln mit sehr kleinem Durchmesser, z.B. größer 23 gauge, bedeutet jedoch auch einen hohen Rohrreibungsbeiwert für das strömende Fluid. Zum Vergleich wird darauf hingewiesen, dass ein Außendurchmesser von 25 gauge einem Außendurchmesser von 0,5 mm entspricht. Je kleiner aber der Durchmesser der Nadel ist, umso größer muss der Druck sein, mit dem das Fluid durch die Rohrleitung bzw. die Nadel transportiert wird, um den gleichen Fluidvolumenstrom zu erreichen. Bei den bekannten ophthalmochirurgischen Systemen reicht dazu der verfügbare Fluiddruck oft nicht aus.

Während einer Operation kann es zudem passieren, dass sehr schnell ein höherer Irrigationsfluiddruck erforderlich ist. Ein Verstellen der Irrigationsfluidquelle nimmt dann zu viel Zeit in Anspruch und/oder kann den erforderlichen Druck nicht bereitstellen, so dass Komplikationen während der Operation unvermeidbar sind.

Es ist daher eine Aufgabe, ein chirurgisches System zur Behandlung von Augenkrankheiten mit der Phakoemulsifikationstechnik zu schaffen, mit dem Operationen durchführbar sind, welche keine postoperative Behandlung mehr erfordern, so dass sehr kleine Hohlnadeln verwendet werden können, wobei gleichzeitig das System in sehr kurzer Zeit einen hohen und gleichzeitig stabilen Fluiddruck zur Verfügung stellen kann.

Die Aufgabe wird durch ein System mit den Merkmalen des unabhängigen Anspruchs 1 gelöst. Vorteilhafte Weiterbildungen der Erfindung sind Gegenstand der Unteransprüche.

Das erfindungsgemäße ophthalmochirurgische System weist auf: Eine erste Irrigationsfluidquelle, eine erste Irrigationsfluidleitung, welche an einem ersten Ende mit der ersten Irrigationsfluidquelle verbunden ist und am anderen Ende offen ist, so dass Fluid von der Irrigationsfluidquelle zu dem offenen Ende und von dort zu einer zu behandelnden Augenlinse transportierbar ist, eine Aspirationsleitung, mit welcher Fluid von der Augenlinse abführbar ist, eine Saugpumpe an welche die Aspirationsfluidleitung angeschlossen ist, eine zweite Irrigationsfluidquelle, an welche eine zweite Irrigationsfluidleitung angeschlossen ist, welche mit der ersten Irrigationsfluidleitung an einer Kopplungsstelle verbunden ist, so dass Irrigationsfluid von der zweiten Irrigationsfluidleitung in die erste Irrigationsfluidleitung zuführbar ist, wobei sich mit der zweiten Irrigationsfluidquelle ein von der ersten Irrigationsfluidquelle höherer Fluiddruck aufbauen lässt, als er maximal bei der ersten Irrigationsfluidquelle erreichbar ist, ein erstes Ventil, welches zwischen dem ersten Ende der ersten Irrigationsfluidleitung und der Kopplungsstelle angeordnet ist, und ein zweites Ventil welches zwischen der zweiten Irrigationsfluidquelle und der Kopplungsstelle angeordnet ist.

Bei diesem System sind zwei Irrigationsfluidquellen vorgesehen, wobei die zweite Irrigationsfluidquelle einen höheren Fluiddruck zur Verfügung stellen kann als die erste Irrigationsfluidquelle. Die Ventile lassen sich jeweils so steuern, dass jeweils nur eine Irrigationsfluidquelle oder beide Irrigationsfluidquellen Fluid in die erste Irrigationsfluidleitung zuführen. Wenn kein hoher Fluiddruck erforderlich ist, kann mit der ersten Irrigationsfluidquelle gearbeitet werden. Bei hohem Fluiddruck kann die zweite Irrigationsfluidquelle dazugeschaltet werden oder alternativ zur ersten Irrigationsfluidquelle eingesetzt werden. Die zweite Irrigationsfluidquelle kann z.B. unter Druck stehendes Fluid aufweisen oder so hoch im Raum angeordnet sein, z.B. direkt unter der Raumdecke, dass ein hoher hydrostatischer Druck vorliegt. Ist während einer Operation ein hoher Fluiddruck erforderlich, ist das erste Ventil so geschaltet, dass das Fluid von der zweiten Irrigationsfluidquelle ausschließlich in Richtung zu der zu behandelnden Augenlinse strömt und nicht in Richtung zur ersten Irrigationsfluidquelle. Durch die Ventile ist auch ein schnelles Umschalten von einem üblichen Druck zu einem hohen Druck einfach realisierbar. Es ist nicht mehr erforderlich, einen Irrigationsfluidbehälter auf eine bestimmte Höhe mit einem Gestänge zu verlagern und abzuwarten, bis Druckschwankungen im System auf ein akzeptables Maß abgeklungen sind. Operationen, bei denen ein normaler und ein hoher Fluiddruck vorgesehen sind, können daher mit höherer Sicherheit durchgeführt werden. Mit einem solchem System können somit relativ große, aber auch sehr kleine Operationsnadeln eines Handstückes bei der Phakoemulsifikation verwendet werden.

Gemäß einer Weiterbildung der Erfindung ist die Saugpumpe eine erste Kolbenpumpe, welche mit einem steuerbaren Antrieb versehen ist, wobei die Kolbenpumpe geeignet ist, aspiriertes Fluid in ihrem Kolbenzylinder aufzunehmen. Im Gegensatz zur üblichen Peristaltikpumpe ermöglicht eine Kolbenpumpe während eines Hubes einen konstanten Volumenstrom bei konstantem Fluiddruck, so dass eine Aspiration mit einer hohen Augenstabilität durchführbar ist. Druckschwankungen bzw. eine ungleichmäßige und stoßartige oder ruckartige Förderung von Fluid, wie sie bei Peristaltikpumpen prinzipiell vorliegen, treten beim Einsatz einer Kolbenpumpe, welche nicht zyklisch und mit hoher Frequenz sondern nur mit einem Hub entlang des Kolbenzylinders bewegt wird, nicht auf. Um einen Abrieb des Kolbens oder des Dichtmittels zwischen Kolben und Zylinderwand so gering wie möglich zu halten, kann die Kolbenpumpe nach einem oder nur wenigen Hüben ausgewechselt werden. Die Kolbenpumpe erlaubt ferner eine sehr genaue Dosierung bei kleinen Fördermengen, so dass auch beim Einsatz von sehr kleinen Nadeln mit nur sehr kleinem Volumenstrom eine zuverlässige Aspiration erreichbar ist.

Vorzugsweise ist ein drittes Ventil in der Aspirationsleitung zwischen der zu behandelnden Augenlinse und der ersten Kolbenpumpe angeordnet. Ein solches Ventil kann zuverlässig verhindern, dass bei Druckschwankungen ein aspiriertes Fluid zurück in Richtung zur Augenlinse fließt.

Die zweite Irrigationsfluidquelle ist vorzugsweise eine zweite Kolbenpumpe, welche mit einem steuerbaren Antrieb versehen ist, wobei die zweite Kolbenpumpe geeignet ist, im Kolben enthaltenes Irrigationsfluid in die zweite Irrigationsfluidleitung abzugeben. Die oben erwähnten Vorzüge der Kolbenpumpe sind gleichermaßen gültig, wenn die Kolbenpumpe für die Irrigation eingesetzt wird. Ein konstanter Volumenstrom bei konstantem Druck in einem geschlossenen System und zusätzlich frei von Druckschwankungen kann somit sehr einfach realisiert werden. Zusätzlich ermöglicht die zweite Kolbenpumpe, dass das Irrigationsfluid mit einem sehr hohen Druck in die erste Irrigationsfluidleitung zuführbar ist.

In einer Ausführungsform der Erfindung kann in der ersten Irrigationsfluidleitung ein viertes Ventil vorgesehen sein, welches zwischen der Kopplungsstelle mit der zweiten Irrigationsfluidleitung und dem offenem Ende der ersten Irrigationsfluidleitung angeordnet ist. Dieses Ventil kann so geschaltet sein, dass zwischen Kopplungsstelle und offenem Ende der ersten Irrigationsfluidleitung kein Fluid mehr fließt, so dass via des ersten Ventils Irrigationsfluid von der ersten Irrigationsfluidquelle in den Behälter der zweiten Irrigationsfluidquelle gefördert werden kann. Beim Einsatz der zweiten Kolbenpumpe kann somit der Kolben in einer Rückbewegung das Fluid in den Kolbenzylinder saugen, so dass das Irrigationsfluid bei einer Hinbewegung des Kolbens herausgedrückt werden kann.

Das erfindungsgemäße ophthalmochirurgische System weist bei einer weiteren Ausführungsform eine dritte Irrigationsfluidquelle auf, welche mittels einer dritten Irrigationsfluidleitung mit der Aspirationsleitung verbunden ist, wobei in der dritten Irrigationsfluidleitung ein fünftes Ventil vorgesehen ist. Falls in der Aspirationsfluidleitung eine Okklusion auftritt, so dass sich bei einer eingeschalteten Saugpumpe in der Aspirationsfluidleitung ein Unterdruck ausbildet, kann in einem Moment des Durchbrechens dieser Okklusion eine relativ hohe Fluidmenge aus dem Auge herausgesaugt werden. Um die Augenkammerstabilität nicht zu gefährden, kann in einem solchen Fall durch die dritte Irrigationsfluidquelle Fluid in die Aspirationsleitung zugeführt werden. Vorzugsweise ist die dritte Irrigationsfluidquelle eine dritte Kolbenpumpe, welche mit einem steuerbaren Antrieb versehen ist, wobei die dritte Kolbenpumpe geeignet ist, im Kolben enthaltenes Irrigationsfluid in die Aspirationsleitung abzugeben. Durch entsprechende Ansteuerung der dritten Kolbenpumpe kann ein großes Fluidvolumen mit hohem Druck schnell zur Verfügung gestellt werden.

Gemäß einer weiteren Ausführungsform der Erfindung ist die dritte Irrigationsfluidquelle mit der ersten Irrigationsfluidquelle mittels einer Befüllungsleitung verbunden, in welcher ein sechstes Ventil angeordnet ist. Ein Druckausgleich in der Aspirationsleitung ist mit entsprechend geschaltetem sechsten Ventil somit derart möglich, dass eine vollständige Trennung von der ersten Irrigationsfluidquelle vorliegt. Daher werden während eines Okklusionsdurchbruches bzw. des während des Belüftens der Aspirationsfluidleitung keine Druckschwankungen in der ersten Irrigationsfluidleitung angeregt, so dass eine hohe Augenkammerstabilität erreichbar ist.

Weitere Vorteile und Merkmale der Erfindung werden mit Bezug auf die nachfolgenden Figuren erklärt, in welchen zeigen:
- Figur 1: eine schematische Darstellung einer ersten Ausführungsform des ophthalmochirurgischen Systems gemäß der Erfindung;
- Figur 2: eine schematische Darstellung einer zweiten Ausführungsform des ophthalmochirurgischen Systems gemäß der Erfindung;
- Figur 3: eine schematische Darstellung einer dritten Ausführungsform des ophthalmochirurgischen Systems gemäß der Erfindung;
- Figur 4: eine schematische Darstellung einer vierter Ausführungsform des ophthalmochirurgischen Systems gemäß der Erfindung;
- Figur 5: eine schematische Darstellung einer fünfter Ausführungsform des ophthalmochirurgischen Systems gemäß der Erfindung; und
- Figur 6: eine schematische Darstellung einer sechster Ausführungsform des ophthalmochirurgischen Systems gemäß der Erfindung,

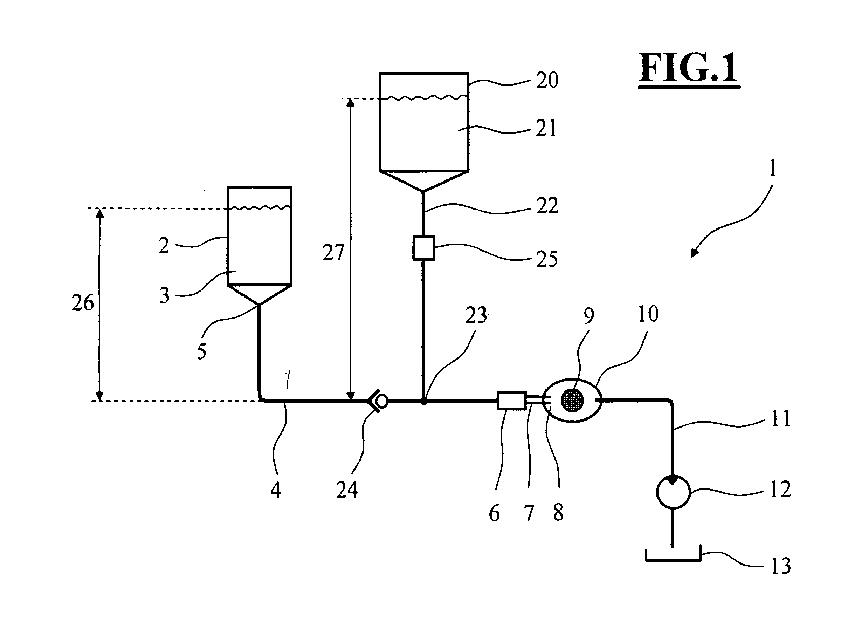

In Figur 1 ist eine schematische Darstellung einer ersten Ausführungsform des erfindungsgemäßen ophthalmochirurgischen Systems 1 gezeigt. In einer ersten Irrigationsfluidquelle 2 ist Irrigationsfluid 3 enthalten, welches durch eine erste Irrigationsfluidleitung 4 von einem ersten Ende 5 in Richtung zu einem Handstück 6 mit einer Nadel 7 und einem dort offenen Ende 8 strömen kann. Bei der Phakoemulsifikation wird die Nadel 7 in die zu behandelnde Augenlinse 9 des Auges 10 eingeführt, wobei die Nadel mit Ultraschall schwingt und die Linse in kleine Partikel emulsifiziert wird. Die Irrigationsfluidquelle 2 ist höher als die Augenlinse 9 angeordnet, so dass das Irrigationsfluid 3 mit einem hydrostatischen Druck 26 in Richtung zur Augenlinse 9 strömen kann. Die emulsifizierten Partikel werden bei der Phakoemulsifikation zusammen mit dem zugeführten Fluid durch eine Aspirationsleitung 11 abtransportiert, an welche eine Saugpumpe 12 angeschlossen ist, welche das abgesaugte Fluid einschließlich emulsifizierter Partikel in einen Sammelbehälter 13 abgeben kann.

Bei dem in Figur 1 dargestellten System 1 ist eine zweite Irrigationsfluidquelle 20 mit Irrigationsfluid 21 mit einer zweiten Irrigationsfluidleitung 22 so vorgesehen, dass die zweite Irrigationsfluidleitung 22 an einer Kopplungsstelle 23 mit der ersten Irrigationsfluidleitung 4 gekoppelt ist. Das Irrigationsfluid 21 kann somit durch die Irrigationsfluidleitung 22 bis zur Kopplungsstelle 23 und von dort über die erste Irrigationsfluidleitung 4 in Richtung zum Handstück 6 bzw. der Nadel 7 strömen. Zwischen dem ersten Ende 5 der ersten Irrigationsleitung 4 und der Kopplungsstelle 23 ist ein erstes Ventil 24 angeordnet, welches dazu dient, den Volumenstrom zwischen der ersten Irrigationsfluidquelle 2 und der Kopplungsstelle 23 zu unterbrechen oder freizugeben. Das erste Ventil kann ein passives oder ein aktiv angesteuertes Ventil sein. Ein zweites Ventil 25 ist zwischen der zweiten Irrigationsfluidquelle 20 und der Kopplungsstelle 23 angeordnet. Dieses zweite Ventil 25 kann so angesteuert werden, dass Irrigationsfluid 21 durch die zweite Irrigationsfluidleitung 22 über die Kopplungsstelle 23 in Richtung zum Handstück 6 und dort in Richtung zur zu behandelnden Augenlinse 9 strömen kann. In einem solchen Fall, bei dem das Irrigationsfluid 21 mit hohem Druck in der zweiten Irrigationsfluidleitung 22 und zwischen der Kopplungsstelle 23 und dem Handstück 6 strömt, muss das erste Ventil 24 geschlossen sein, so dass sichergestellt ist, dass das Fluid 21 nicht zur ersten Irrigationsfluidquelle 2 strömt. Der von der zweiten Irrigationsfluidquelle 20 bereitgestellte relativ hohe Fluiddruck kann zum Beispiel derart erreicht werden, dass die zweite Irrigationsfluidquelle 20 höher als die erste Irrigationsfluidquelle 2 angeordnet ist, so dass im Vergleich zum hydrostatischen Druck 26 ein höherer hydrostatischer Druck 27 vorliegt. Das Irrigationsfluid 21 kann aber zum Beispiel auch unter einem relativ hohen Behälterdruck innerhalb der zweiten Irrigationsfluidquelle vorliegen, so dass auch bei gleicher Höhe im Vergleich zur ersten Irrigationsfluidquelle 2 ein höherer Fluiddruck erreichbar ist.

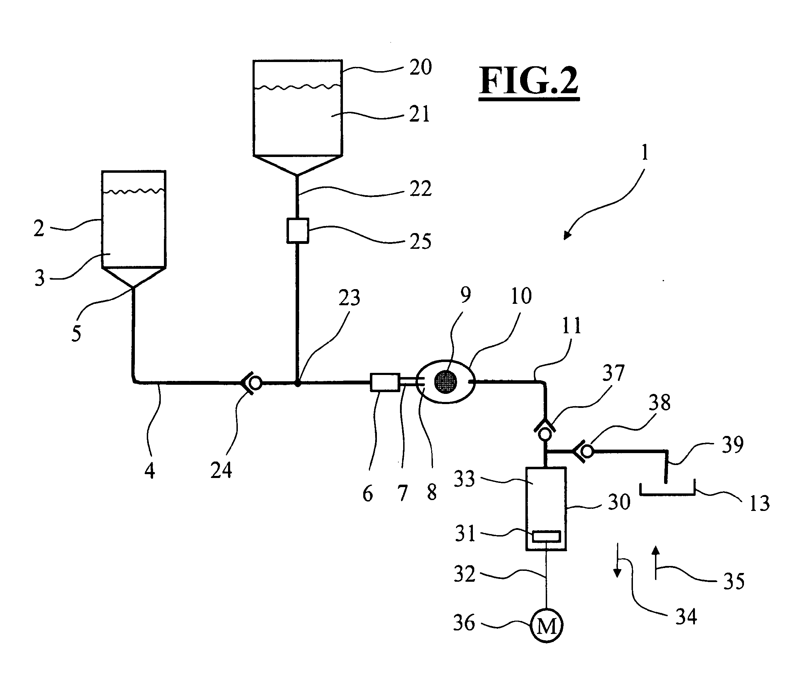

Wie aus Figur 2 ersichtlich ist, kann bei einer Ausführungsform der Erfindung die zweite Irrigationsfluidquelle 20 als Kolbenpumpe 30 ausgebildet sein, welche einen Kolben 31 mit einer Kolbenstange 32 aufweist, die in einem Kolbenzylinder 33 verlagerbar sind. Wird der Kolben 31 via der Kolbenstange 32 mittels eines Antriebes 36 zurückbewegt, siehe Pfeil 34, nimmt der Kolbenzylinder 33 aspiriertes Fluid und Partikel auf. Druckschwankungen, wie sie bei einer Peristaltikpumpe üblich sind, lassen sich bei Einsatz einer solchen Kolbenpumpe zuverlässig vermeiden, wenn der Kolben 31 während einer Operation nur einmal die Rückbewegung, siehe Pfeil 34, durchführt. Eine Hinbewegung, siehe Pfeil 35, darf der Kolben 31 nicht ausführen, da sonst aspiriertes Fluid in das Auge zurücktransportiert würde. Um dies zuverlässig zu vermeiden, kann mit einem Ventil 37 die Fluidströmung zum Auge hin unterbrochen werden. Hat sich der Kolben 33 mit Fluid und Partikeln gefällt, kann er z.B. vollständig gegen eine noch nicht gefüllte Kolbenpumpe ersetzt werden. Eine andere Möglichkeit besteht darin, nach Schließen des Ventils 37 den Kolben 32 so anzutreiben, dass er in einer Hinbewegung, siehe Pfeil 35, das Fluid aus dem Kolben 33 in eine Abführleitung 39 mit Ventil 38 und von dort zu einem Sammelbehälter 13 zu befördern. Anschließend kann der Kolben 33 in einer Rückbewegung wieder aspiriertes Fluid aufnehmen.

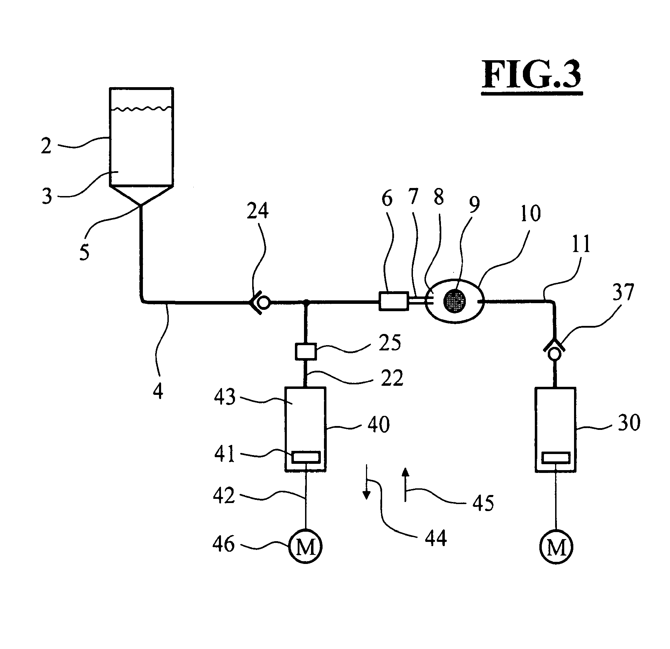

In Figur 3 ist eine dritte Ausführungsform der Erfindung schematisch dargestellt. Die Irrigationsfluidquelle 20 ist hierbei eine zweite Kolbenpumpe 40 mit einem Kolben 41 und einer Kolbenstange 42, welche sich in einem Kolbenzylinder 43 mittels eines Antriebes 46 verlagern lassen. In einer Hinbewegung, siehe Pfeil 45, kann der Kolben 41 Fluid im Kolbenzylinder 43 in die zweite Irrigationsfluidleitung 22 befördern. Das Ventil 25 gibt den Volumenstrom in Richtung zur ersten Irrigationsfluidleitung 4 frei oder sperrt diesen.

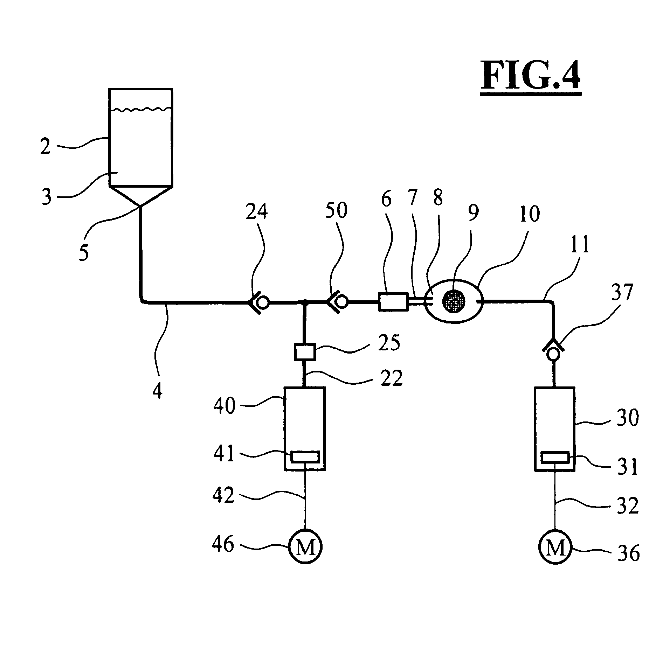

Falls in der ersten Irrigationsfluidleitung 4 ein Ventil 50 vorgesehen ist, siehe Figur 4, kann dieses so geschaltet sein, dass bei einer Rückbewegung, siehe Pfeil 44, des Kolbens 41 Fluid von der ersten Irrigationsfluidquelle 2 durch die erste Irrigationsfluidleitung 4, das erste Ventil 24, die zweite Irrigationsfluidleitung 22 und durch das dritte Ventil 25 fließt. Damit kann die zweite Irrigationsfluidquelle 20 bzw. 40 befüllt werden.

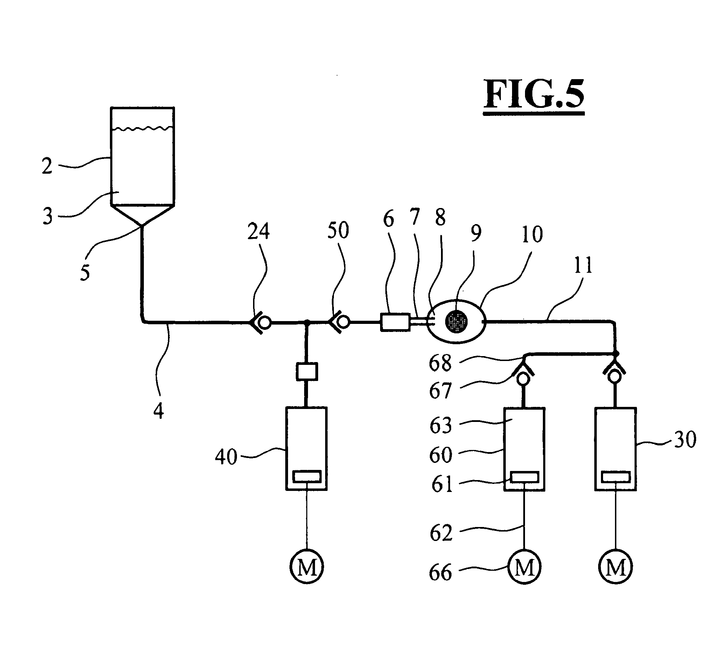

Falls während einer Aspiration eine Okklussion bzw. eine Verstopfung der Aspirationsfluidleitung 11 auftritt, kann sich in dieser Leitung 11 ein Unterdruck aufbauen. Bricht die Verstopfung auf, entsteht ein gefährlicher Sog, welcher zuviel Fluid aus dem Augeninnenraum heraussaugen kann. Um dies zu verhindern, siehe Figur 5, kann eine dritte Irrigationsfluidquelle 60 mit einem kolben 61, einer Kolbenstange 62 und einem Antrieb 66 vorgesehen sein, welche mittels eines fünften Ventils 67 in Abhängigkeit von einem vorliegenden Unterdruck in der Aspirationsfluidleitung 11 Irrigationsfluid 63 über eine dritte Irrigationsfluidleitung 68 zur Aspirationsfluidleitung 11 zuführt. Damit lässt sich ein Kollaps des Augeninnenraumes zuverlässig verhindern.

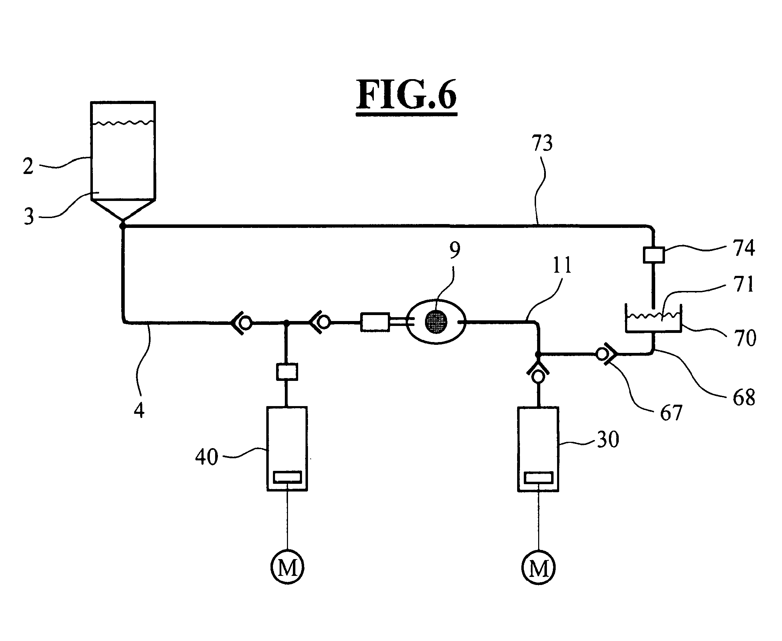

Wie aus Figur 6 ersichtlich ist, kann bei einer weiteren Ausführungsform der Erfindung die dritte Irrigationsfluidquelle als ein Behälter 70 ausgebildet sein, welcher das darin enthaltene Irrigationsfluid 71 über die dritte Irrigationsfluidleitung 68 zur Aspirationsfluidleitung 11 zuführen kann. Die dritte Irrigationsfluidquelle 70 ist bei dieser Ausführungsform mittels einer Befüllungsleitung 73 und einem sechsten Ventil 74 mit der ersten Irrigationsfluidquelle 2 verbunden. Im Falle eine Okklussionsdurchbruches in der Aspiratonsfluidleitung 11 wird damit eine Druckschwankung in der ersten Irrigationsleitung 4 zuverlässig verhindert.

## Patentansprüche

1. Ophthalmochirurgisches System (1), aufweisend:
- eine erste Irrigationsfluidquelle (2)
- eine erste Irrigationsfluidleitung (4), welche an einem ersten Ende (5) mit der ersten Irrigationsfluidquelle (2) verbunden ist und am anderen Ende (8) offen ist, so dass Fluid (3) von der Irrigationsfluidquelle (2) zu dem offenen Ende (8) und von dort zu einer zu behandelnden Augenlinse (9) transportierbar ist,
- eine Aspirationsfluidleitung (11), mit welcher Fluid von der Augenlinse (9) abführbar ist,
- eine Saugpumpe (12; 30), an welche die Aspirationsfluidleitung angeschlossen ist,
**dadurch gekennzeichnet, dass**
- eine zweite Irrigationsfluidquelle (20; 40) vorgesehen ist,
- eine zweite Irrigationsfluidleitung an die zweite Irrigationsfluidquelle (20;40) angeschlossen ist, welche mit der ersten Irrigationsfluidleitung (4) an einer Kopplungsstelle (23) verbunden ist, so dass Irrigationsfluid (21) von der zweiten Irrigationsfluidleitung (22) in die erste Irrigationsfluidleitung (4) zuführbar ist, wobei sich mit der zweiten Irrigationsfluidquelle (20; 40) ein von der ersten Irrigationsfluidquelle (2) höherer Fluiddruck aufbauen lässt, als er maximal bei der ersten Irrigationsfluidquelle (2) erreichbar ist, und
- ein erstes Ventil (24) zwischen dem ersten Ende (5) der ersten Irrigationsfluidleitung (4) und der Kopplungsstelle (23) angeordnet ist und
- ein zweites Ventil (25) zwischen der zweiten Irrigationsfluidquelle (20;40) und der Kopplungsstelle (23) angeordnet ist.

2. System (1) nach Anspruch 1, wobei die Saugpumpe (12) eine erste Kolbenpumpe (30) ist, welche mit einem steuerbaren Antrieb (36) versehen ist, wobei die Kolbenpumpe (30) geeignet ist, aspiriertes Fluid in ihrem Kolbenzylinder (33) aufzunehmen.

3. System (1) nach einem der Ansprüche 1 oder 2, wobei ein drittes Ventil (37) in der Aspirationsleitung (11) zwischen der zu behandelnden Augenlinse (9) und der ersten Saugpumpe (12; 30) angeordnet ist.

4. System (1) nach einem der Ansprüche 1 bis 3, wobei die zweite Irrigationsfluidquelle (20) eine zweite Kolbenpumpe (40) ist, welche mit einem steuerbaren Antrieb (46) versehen ist, wobei die zweite Kolbenpumpe (40) geeignet ist, im Kolben (41) enthaltenes Irrigationsfluid in die zweite Irrigationsfluidleitung (22) abzugeben.

5. System (1) nach einem der Ansprüche 1 bis 4, wobei in der ersten Irrigationsfluidleitung (4) ein viertes Ventil (50) vorgesehen ist, welches zwischen der Kopplungsstelle (23) und dem offenen Ende (8) der ersten Irrigationsfluidleitung (4) angeordnet ist.

6. System (1) nach einem der Ansprüche 1 bis 5, wobei das System (1) eine dritte Irrigationsfluidquelle (60) aufweist, welche mittels einer dritten Irrigationsfluidleitung (68) mit der Aspirationsfluidleitung (11) verbunden ist, wobei in der dritten Irrigationsfluidleitung (68) ein fünftes Ventil (67) vorgesehen ist.

7. System (1) nach Anspruch 6, wobei die dritte Irrigationsfluidquelle (60) eine dritte Kolbenpumpe (60) ist, welche mit einem steuerbaren Antrieb (66) versehen ist, wobei die dritte Kolbenpumpe (60) geeignet ist, im Kolben (63) enthaltenes Irrigationsfluid mittels der dritten Irrigationsfluidleitung (68) in die Aspirationsleitung (11) abzugeben.

8. System (1) nach einem der Ansprüche 6 oder 7, wobei die dritte Irrigationsfluidquelle (60; 70) mit der ersten Irrigationsfluidquelle (2) mittels einer Befüllungsleitung (73) verbunden ist, in welcher ein sechstes Ventil (74) angeordnet ist.

## Claims

1. Ophthalmosurgical system (1), comprising:
- a first irrigation fluid source (2),
- a first irrigation fluid line (4), which is connected to the first irrigation fluid source (2) at a first end (5) and is open at the other end (8) such that fluid (3) can be transported from the irrigation fluid source (2) to the open end (8) and from there to an ocular lens (9) to be treated,
- an aspiration fluid line (11) by means of which fluid can be removed from the ocular lens (9),
- a suction pump (12; 30), to which the aspiration fluid line is connected,
**characterized in that**
- a second irrigation fluid source (20; 40) is provided,
- a second irrigation fluid line is connected to the second irrigation fluid source (20; 40) and connected to the first irrigation fluid line (4) at a coupling site (23), so that irrigation fluid (21) can be supplied into the first irrigation fluid line (4) from the second irrigation fluid line (22), wherein a fluid pressure from the first irrigation fluid source (2) can be built up using the second irrigation fluid source (20; 40), which fluid pressure is higher than can be achieved at most in the case of the first irrigation fluid source (2), and
- a first valve (24) is arranged between the first end (5) of the first irrigation fluid line (4) and the coupling site (23), and
- a second valve (25) is arranged between the second irrigation fluid source (20; 40) and the coupling site (23).

2. System (1) according to Claim 1, wherein the suction pump (12) is a first piston pump (30), which is provided with a controllable drive (36), wherein the piston pump (30) is suitable for holding aspirated fluid in the piston cylinder (33) thereof.

3. System (1) according to either Claim 1 or 2, wherein a third valve (37) is arranged in the aspiration line (11) between the ocular lens (9) to be treated and the first suction pump (12; 30).

4. System (1) according to one of Claims 1 to 3, wherein the second irrigation fluid source (20) is a second piston pump (40), which is provided with a controllable drive (46), wherein the second piston pump (40) is suitable for dispensing irrigation fluid contained in the piston (41) into the second irrigation fluid line (22).

5. System (1) according to one of Claims 1 to 4, wherein a fourth valve (50) is provided in the first irrigation fluid line (4), which valve is arranged between the coupling site (23) and the open end (8) of the first irrigation fluid line (4).

6. System (1) according to one of Claims 1 to 5, wherein the system (1) has a third irrigation fluid source (60), which is connected to the aspiration fluid line (11) by means of a third irrigation fluid line (68), wherein a fifth valve (67) is provided in the third irrigation fluid line (68).

7. System (1) according to Claim 6, wherein the third irrigation fluid source (60) is a third piston pump (60), which is provided with a controllable drive (66), wherein the third piston pump (60) is suitable for dispensing irrigation fluid contained in the piston (63) into the aspiration line (11) by means of the third irrigation fluid line (68).

8. System (1) according to either Claim 6 or 7, wherein the third irrigation fluid source (60; 70) is connected to the first irrigation fluid source (2) by means of a filling line (73), in which a sixth valve (74) is arranged.

## Revendications

1. Système de chirurgie ophtalmique (1), comprenant :
- une première source de fluide d'irrigation (2),
- une première ligne de fluide d'irrigation (4) qui est reliée au niveau d'une première extrémité (5) à la première source de fluide d'irrigation (2) et ouverte à l'autre extrémité (8) de telle sorte que du fluide (3) provenant de la source de fluide d'irrigation (2) peut être transporté vers l'extrémité ouverte (8) et de là vers une lentille ophtalmique (9) à traiter,
- une ligne de fluide d'aspiration (11) permettant d'évacuer du fluide provenant de la lentille ophtalmique (9),
- une pompe aspirante (12 ; 30) à laquelle est raccordée la ligne de fluide d'aspiration,
**caractérisé en ce**
- **qu'**une deuxième source de fluide d'irrigation (20 ; 40) est prévue,
- une deuxième ligne de fluide d'irrigation est raccordée à la deuxième source de fluide d'irrigation (20 ; 40), qui est reliée à la première ligne de fluide d'irrigation (4) en un point d'accouplement (23) de telle sorte que du fluide d'irrigation (21) peut être amené de la deuxième ligne de fluide d'irrigation (22) à la première ligne de fluide d'irrigation (4), la deuxième source de fluide d'irrigation (20 ; 40) permettant d'établir une pression de fluide à partir de la première source de fluide d'irrigation (2) supérieure à celle qui peut être atteinte au maximum au niveau de la première source de fluide d'irrigation (2), et
- une première vanne (24) est disposée entre la première extrémité (5) de la première ligne de fluide d'irrigation (4) et le point d'accouplement (23), et
- une deuxième vanne (25) est disposée entre la deuxième source de fluide d'irrigation (20 ; 40) et le point d'accouplement (23).

2. Système (1) selon la revendication 1, dans lequel la pompe aspirante (12) est une première pompe à piston (30) qui est munie d'un dispositif d'entraînement réglable (36), dans lequel la pompe à piston (30) est adaptée pour recevoir du fluide aspiré dans son cylindre de piston (33).

3. Système (1) selon l'une quelconque des revendications 1 ou 2, dans lequel une troisième vanne (37) est disposée sur la ligne d'aspiration (11) entre la lentille ophtalmique (9) à traiter et la première pompe aspirante (12 ; 30).

4. Système (1) selon l'une quelconque des revendications 1 à 3, dans lequel la deuxième source de fluide d'irrigation (20) est une deuxième pompe à piston (40) qui est munie d'un dispositif d'entraînement réglable (46), dans lequel la deuxième pompe à piston (40) est adaptée pour délivrer à la deuxième ligne de fluide d'irrigation (22) du fluide d'irrigation contenu dans le piston (41).

5. Système (1) selon l'une quelconque des revendications 1 à 4, dans lequel sur la première ligne de fluide d'irrigation (4) est prévue une quatrième vanne (50) qui est disposée entre le point d'accouplement (23) et l'extrémité ouverte (8) de la première ligne de fluide d'irrigation (4).

6. Système (1) selon l'une quelconque des revendications 1 à 5, dans lequel le système (1) présente une troisième source de fluide d'irrigation (60) qui est reliée au moyen d'une troisième ligne de fluide d'irrigation (68) à la ligne de fluide d'aspiration (11), dans lequel une cinquième vanne (67) est prévue sur la troisième ligne de fluide d'irrigation (68).

7. Système (1) selon la revendication 6, dans lequel la troisième source de fluide d'irrigation (60) est une troisième pompe à piston (60) qui est munie d'un dispositif d'entraînement réglable (66), dans lequel la troisième pompe à piston (60) est adaptée pour délivrer à la ligne d'aspiration (11) du fluide d'irrigation contenu dans le piston (63), au moyen de la troisième ligne de fluide d'irrigation (68).

8. Système (1) selon l'une quelconque des revendications 6 ou 7, dans lequel la troisième source de fluide d'irrigation (60 ; 70) est reliée à la première source de fluide d'irrigation (2) au moyen d'une ligne de remplissage (73) sur laquelle est disposée une sixième vanne (74).
